# EUROPÄISCHE PATENTSCHRIFT

(11) **EP 3 160 381 B1**
(45) Veröffentlichungstag und Bekanntmachung des Hinweises auf die Patenterteilung: **29.08.2018**
(21) Anmeldenummer: 15733663.7
(22) Anmeldetag: 22.06.2015
(51) Int. Cl.: A61C 5/90, A61B 1/24, A61B 13/00

(54) **FOLIENSPANNELEMENT**
FILM CLAMPING ELEMENT
ÉLÉMENT DE TENSION DE FEUILLE

(30) Priorität: 26.06.2014 DE 102014109023
(43) Veröffentlichungstag der Anmeldung: 03.05.2017
(73) Patentinhaber: Ivoclar Vivadent AG, 9494 Schaan (LI)
(72) Erfinder: MÜLLER, Frank, A-6800 Feldkirch (AT); ENGGIST, Lukas, CH-7320 Sargans (CH); WALTHER, Nora, CH-9450 Altstätten (CH)
(74) Vertreter: Baronetzky, Klaus
(86) Internationale Anmeldenummer: PCT/EP2015/063995
(87) Internationale Veröffentlichungsnummer: WO 2015/197560

(56) Entgegenhaltungen:
- WO-A1-03/051185
- DE-U1- 29 702 483
- US-A- 5 524 644

## Beschreibung

Die Erfindung betrifft ein Folienspannelement. In diesem Zusammenhang wird auf die WO 03/051185 A1 verwiesen. Ein Folienspannelement gemäß dem Oberbegriff von Anspruch 1 wird seit knapp zehn Jahren erfolgreich eingesetzt, um den freien Zugang zum Patientenmund bei dentalen Bearbeitungen im Mundraum eines Patienten zu ermöglichen. Es besteht aus einem Vestibulärring und einem Lippenring, zwischen denen sich eine Folie erstreckt. Bei diesem Element ist die Folie gegenüber beiden Ringen verschieblich gelagert und elastisch. Zur Vermeidung von allergischen Beeinträchtigungen ist sie latexfrei ausgebildet.

Durch das Umschlagen der Folie soll sichergestellt sein, dass sie selbsttätig hält.

Bei Fehlbedienung kann es aber sein, dass sie über den etwas kleineren Vestibulärring rutscht, so dass die Funktion des Folienspannelements insofern entfallen kann.

Daher liegt der Erfindung die Aufgabe zugrunde, ein verbessertes und gut handhabbares Folienspannelement gemäß dem Oberbegriff von Anspruch 1 zu schaffen, dass zudem mundverträglicher ist.

Diese Aufgabe wird erfindungsgemäß durch Anspruch 1 gelöst. Vorteilhafte Weiterbildungen ergeben sich aus den Unteransprüchen.

Erfindungsgemäß ist vorgesehen, dass die Folie an dem Vestibulärring einen nicht exakt gleichförmigen Verlauf aufweist, sondern einen Rücksprung an bestimmten und eher schmalen Stellen aufweist. Beispielsweise kann der Bereich des Frenulums ausgespart sein und die das Frenulum umgebenden Bereiche des Vestibulärring mit einer Polsterung versehen sein. Die Polsterung kann dann zugleich auch der Fixierung der Folie an dieser Stelle dienen, was es ermöglicht zu verhindern, dass die Folie sich versehentlich über den Vestibulärring zieht und kollabiert.

Dennoch ist durch die Spannung des Folie über den - in der Regel größeren - Lippenring die dreidimensionale Anpassbarkeit und Verformbarkeit überraschend ohne weiteres gegeben, und die erfindungsgemäße Abhaltefunktion ist sicher gewährleistet. Die Polsterung seitlich der Frenuli kann als Verdickung der Folie ausgebildet sein, die über den Vestibulärring gezogen ist und dort ein angenehmeres Tragegefühl im Vergleich mit dem eigentlichen Vestibulärring bietet. Der Mund des Patienten wird im Vestibulum sanft aufgedrückt, ohne dass der Patient das Gefühl einer erzwungenen Mundsperre hat.

Erfindungsgemäß werden gezielt die Lippen, die an der Oberseite und der Unterseite der Folie anliegenden mit einer Spannkraft beaufschlagt, und aufgrund der weichen Polsterauflage im Vestibulum wird diese Spannkraft überraschend vom Patienten als wenig unangenehm empfunden. Demgegenüber ist die Spannkraft der Folie im Bereich der Wangen und Mundwinkel wesentlich geringer, beispielsweise um eine Größenordnung.

Erfindungsgemäß ist durch die Kombination dieser Maßnahmen der Tragekomfort gegenüber den bislang bekannten Lösungen insofern trotz der Verwendung eines im wesentlichen gleich elastischen Vestibulärrings deutlich verbessert, und zusätzlich entfällt die Gefahr, dass die Folie vom Vestibulärring abrutscht.

Aufgrund der Zusatzelastizität im Bereich der Polsterung ist zudem die Belastung des elastischen Vestibulärring im Sinne eines Knickschutzes geringer; die Nachgiebigkeit der Polsterung insofern ist erfindungsgemäß größer als die Nachgiebigkeit des eigentlichen Vestibulärrings.

In erfindunsgemäß bevorzugter Ausgestaltung kann der eigentliche Vestibulärring im Bereich der Frenuli, also an einander gegenüberliegenden Stellen, radial einwärts gebogen verformt sein. In modifizierter Ausgestaltung reicht es aber aus, seitlich der Rücksprungbereiche Vorsprünge auszubilden, die elastisch und/oder plastisch verformbar ausgebildet sein können.

Verschiedene Arten der Polsterung sind erfindungsgemäß möglich. So lassen sich durch Wärmeverformbarkeit oder Feuchtigkeitsverformbarkeit die physiologischen Geggebenheiten in der Mundhöhle, also Feuchtigkeit und/oder Wärme, ausnutzen, um die erwünschte Verformbarkeit erst bei Anwendung der Erfindung zu aktivieren. Es ist auch möglich, die Polsterung mit einer Membran auszustatten, die einseitig in einer Richtung von Flüssigkeit durchtretbar ist und der Gegenrichtung sperrt. Hierdurch wird eine Flüssigkeitsabsorbtion gewährleistet, die zugleich auch die Polsterung des Vestibulärrings gewährleistet, beispielsweise, indem Absorptionskörper innerhalb der Membran angeordnet sind. Diese polstern den etwas härteren eigentlichen Vestibulärring dann nach Außen ab.

Es ist auch möglich, die Polsterbereiche als Hohlkörper auszubilden und/oder diese sich lediglich an den druckrelevanten Stellen erstrecken zu lassen. Beispielsweise können entsprechende Polsterkörper sich im Abstand von etwa 1cm von der zentralen Mitte - die den Bereichen der Frenuli entspricht - nach Außen erstrecken, beispielsweise über 1cm bis 3cm, bevorzugt etwa über 2cm.

Die Lateralbereiche des Vestibulärrings sind dann frei von Polsterkörpern und insofern noch etwas elastisches als die Polsterbereiche.

Die Hohlkörper können dann entweder mit Flüssigkeit wie Wasser, mit Luft oder auch mit einem saugfähigen Material wie einem Schwamm gefüllt sein, und können sich entweder durch den Bediener betätigt auf einen gewünschten Innendruck einstellen lassen oder von vorneherein mit einem vorgegebenen Innendruck vorgestellt sein. Bei Flüssigkeitsfüllung lässt sich auch der Wasserbetteffekt realisieren, indem der jeweilige Hohlkörper nur teilgefüllt ist. In diesem Fall erfolgt eine optimale und zugleich kühlende Anpassung an die Anlagefläche im Vestibulum.

Bei einer unrunden Ausgestaltung des eigentlichen Vestibulärrings lassen sich zudem durch exakte Festlegung der Steifigkeit an unterschiedlichen radialen Stellen des Vestibulärrings die erwünschten Rückstellkräfte einstellen, und es ist auch möglich, zusätzlich zur elastischen Verformbarkeit eine plastische Teilverformbarkeit zu realisieren.

Der Vestibulärring kann in einer modifizierten Ausgestaltung auch oval abgeflacht vorgeformt sein.

Weitere Vorteile, Einzelheiten und Merkmale der Erfindung ergeben sich aus der nachfolgenden Beschreibung mehrerer Ausführungsbeispiele der Erfindung anhand der Zeichnungen.

Es zeigen:
- Fig. 1: eine perspektivische Ansicht einer Ausführungsform eines Folienspannelements;
- Fig. 2: eine weitere Ausführungsform eines erfindungsgemäßen Folienspannelements, ebenfalls in perspektivischer Darstellung;
- Fig. 3: eine dritte Ausführungsform eines erfindungsgemäßen Folienspannelements in perspektivischer Darstellung;
- Fig. 4: eine Darstellung eines Details eines erfindungsgemäßen Folienspannelements gemäß einer weiteren Ausführungsform;
- Fig. 5: eine perspektivische Darstellung einer weiteren Ausführungsform eines erfindungsgemäßen Folienspannelements;
- Fig. 6: eine weitere Ausführungsform eines erfindungsgemäßen Folienspannelements, jedoch in schematischer Seitendarstellung.
- Fig. 7: eine Seitenansicht einer weiteren Ausführungsform eines erfindungsgemäßen Folienspannelements; und
- Fig. 8: eine Draufsicht auf eine weitere Ausführungsform eines erfindungsgemäßen Folienspannelements;
- Fig. 9: eine Schnittansicht eines Details einer weiteren Ausführungsform eines erfindungsgemäßen Folienspannelements; und
- Fig. 10: eine Draufsicht auf eine weitere Ausführungsform eines erfindungsgemäßen Folienspannelements.

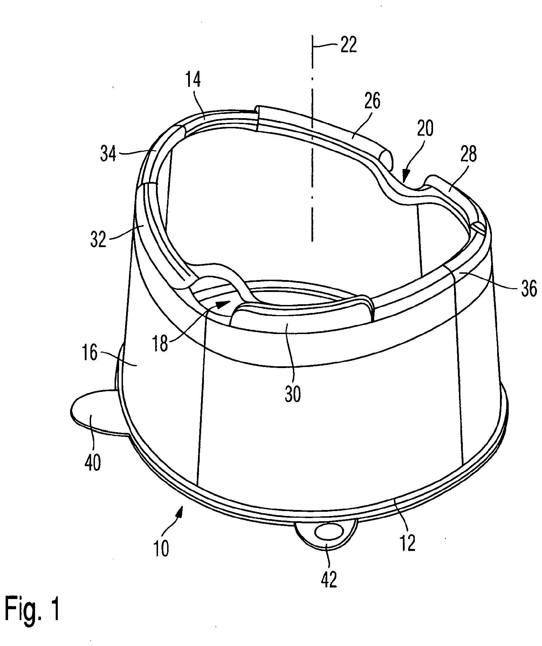

Das Folienspannelement 10 gemäß Fig. 1 weist einen Lippenring 12 und einen Vestibulärring 14 auf. Zwischen diesen ist eine Folie 16, die in dem dargestellten Ausführungsbeispiel gemäß Fig. 1 sowohl am Vestibulärring 14 als auch am Lippenring 12 befestigt ist.

In einer modifizierten Ausgestaltung ist die Folie 16 an dem Vestibulärring 14 fest gelagert, jedoch in an sich bekannter Weise über den Lippenring 12 geschlagen, so dass eine dreidimensionale Verschiebbarkeit und Verformbarkeit insofern besteht.

Aus Fig. 1 ist ersichtlich, dass die Form des Lippenrings 12 und die Form des Vestibulärrings 14 von der Kreisform deutlich abweicht und gegenüber dieser oval/dreieckig verformt ist, In beiden Fällen handelt es sich um eine geschlossene Ringform.

Die Folie 16 ist in dieser Ausführungform im Grunde biegeschlaff. Ohne Zugkraft zwischen dem Vestibulärring 14 und dem Lippenring 12 würde sie daher kollabieren. Erfindungsgemäß ist eine Art weiches Stützkorsett vorgesehen, das in die Folie 16 eingearbeitet ist und die Ringe 12 und 14 auf Abstand hält bzw. die Folie 16 in der Zielform aufgespannt. Dieses Stützkorsett ist so weich bemessen, dass bei Druck mit den Lippen auf die Folie diese sich ohne weiteres und ohne nennenswerten Widerstand lippenfreundlich verformt und damit den Vestibulärring 14 und den Lippenring 12 unter Verformung der Folie 16 etwas aufeinander zu bewegt, oder die Folie bereits in der Zielform hält.

Der Vestibulärring 14 ist erfindungsgemäß in besonderer Weise ausgestaltet. Er weist zwei Rücksprünge 18 und 20 auf. Diese liegen einander gegenüber und sind dafür bestimmt, in das Vestibulum in den Stellen eingeführt zu werden, an denen sich die Frenuli erstrecken. Dort ist der Vestibulärring 14 zu einer Achse 22 des Folienspannelements 10 hin sich erstreckend eingebogen, so dass verhindert wird, dass der harte Teil des Vestibulärrings 14 mit den Frenuli in Kontakt gerät. Das Rücksprungmaß beträgt dementsprechend mindestens mehrere Millimeter, beispielsweise 5mm.

Um die Wirkung des Rücksprungs insofern noch zu verstärken und den Tragekomfort zu verbessern sind seitlich der Rücksprünge 18 und 20, Vorsprünge 26, 28, 30 und 32 vorgesehen. Diese Vorsprünge 26 bis 30 dienen als Polster des Vestibulärrings 14. Sie sind aus einem weicheren Material als der eigentliche Vestibulärring 14 und umgeben diesen mindestens in radial auswärtiger Richtung und in achsparalleler Richtung, bezogen auf die Achse 22. Dies ist aus Fig. 1 ersichtlich.

Die Vorsprünge 26 bis 32 dienen als Polster und springen ebenfalls gegenüber dem eigentlichen Vestibulärring um einiges vor, beispielsweise um 3mm.

Sie erstrecken sich über ein paar Zentimeter seitlich des Rücksprungbereichs 18 und 20, lassen jedoch die Lateralbereiche 34, 36 frei, so dass diese ungehindert einfedern können.

Der Federwiderstand des Vestibulärrings 14 ist daher in den Lateralbereichen 34 und 36 am geringsten und sowohl im Bereich der Polster 26 bis 32 als auch im Bereich der Rücksprünge 18 und 20 höher als im Lateralbereich.

Die elliptische oder leicht dreieckige Form des Vestibulärrings 14 im entspannten Zustand erlaubt eine weiche und elastische Verformung beim Einsetzen des Vestibulärrings 14 in das Vestibulum. Durch die Polster 26 bis 32 ist der Tragekomfort deutlich erhöht, und auch dann, wenn der Patient aufgrund der Einführung eines Fremdkörpers in seinen Mund den betreffenden Bereich mit der Zunge bearbeitet, trennt sich die Folie 16 nicht von dem Vestibulärring 14.

Der Lippenring 12 ist mit Orientierungsvorsprüngen 40 und 42 versehen. Diese sollen dem Oberkiefer des Patienten zugeordnet sein, und können beispielsweise mit Identifikations- oder Herstellerzeichen versehen sein. Sie erstrecken sich quer zur Achse 22 von dem Lippenring 12 nach außen hin und sind in den Ausführungsformen vorgesehen, in denen die Folie 16 nicht über den Lippenring 12 geschlagen und gegenüber diesen beweglich ist, sondern an ihm befestigt ist.

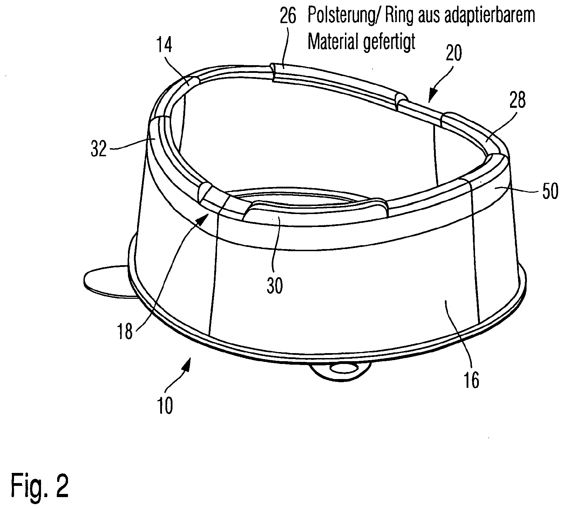

Eine weitere Ausführungsform des erfindungsgemäßen Folienspannelements 10 ist aus Figur 2 ersichtlich. Dort erstreckt sich ein integrierter Vestibulärring 14aus einem einheitlichen Material, in welches die erwünschte Profilierung eingearbeitet ist. Das dort verwendete Material ist weich und umgibt die Hartkomponente des Vestibulärrings 14 vollständig. Dieses Gesamtpolster 50 vermeidet einen Kontakt zwischen harten Materialien des Vestibulärrings 14 und dem Vestibulum des Patienten andererseits, erlaubt aber dennoch die Realisierung von Rücksprüngen 18 und 20 und Vorsprüngen 26 bis 32. Das Polstermaterial des Polsters 50 kann in beliebiger geeigneter Weise durch Anspritzen an den Vestibulärring 14 oder die Folie 16 angebracht sein.

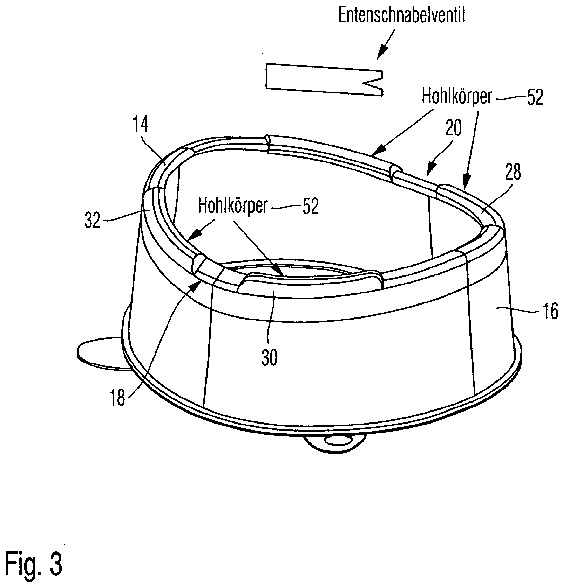

Bei der Ausführungsform gemäß Fig. 3 sind als Polster 26 bis 32 Hohlkörper 52 vorgesehen. Die Hohlkörper 52 sind in diesem Ausführungsbeispiel aufblasbar, beispielsweise über eine kleine Handpumpe mit einem Entenschnabelventil. Hiermit ist der Tragekomfort des Vestibulärrings 14 weiter erhöht.

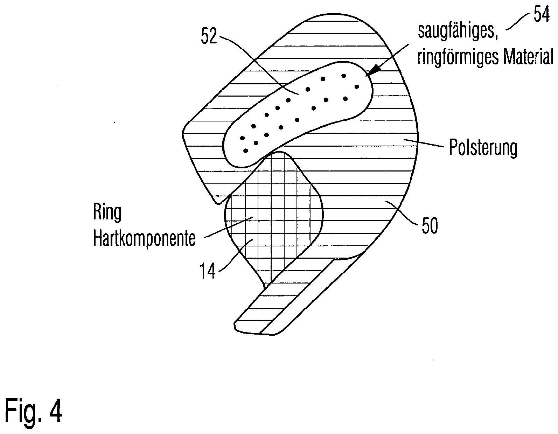

Aus Fig. 4 ist ersichtlich, in welcher Weise ein Hohlkörper 52 an dem Vestibulärring 14 ebenfalls ausgebildet sein kann. Der Vestibulärring 14 ist an drei Seiten von dem Polstermaterial 50 umgeben. Dieses umschließt seinerseits einen Hohlraum 52.

In dem dargestellten Ausführungsbeispiel ist der Hohlraum 52 mit saugfähigem Material gefüllt, beispielsweise einem Schwamm. Bei Flüssigkeitszufuhr, die auch im Mund des Patienten automatisch erfolgen kann, quillt das saugfähige Material 54 auf und erhöht insofern die Polsterwirkung zum Vestibulärring 14 hin.

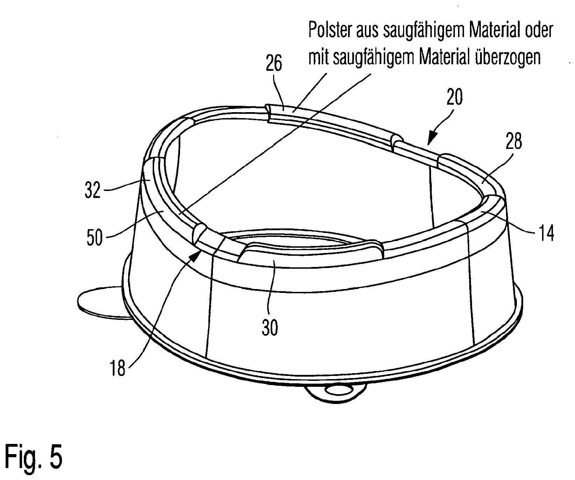

Bei der Ausführungsform gemäß Fig. 5 ist der Vestibulärring 14 an den vier Stellen 26 bis 32, die seitlich von den Rücksprüngen 18 und 20 beabstandet und diesen benachbart sind, mit saugfähigem Material überzogen. Bei Flüssigkeitskontakt erhöht dieses automatisch sein Volumen und passt sich zudem weich federnd der Umgebung, also dem Vestibulum, an.

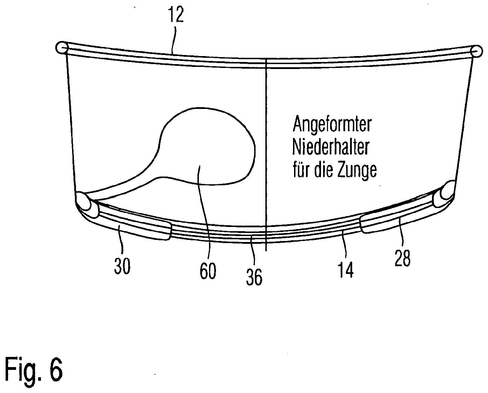

Bei der Ausführungsform gemäß Fig. 6 ist es vorgesehen, einen Niederhalter 60 an dem Vestibulärring 14 anzuformen. Dieser ermöglicht es, die Zunge des Patienten während der Bearbeitung zu fixieren und vom Arbeitsbereich fernzuhalten.

Gleiche Bezugszeichen weisen hier wie auch in den weiteren Figuren auf gleiche oder entsprechende Teile hin, so dass eine zusätzliche Erläuterung entbehrlich ist.

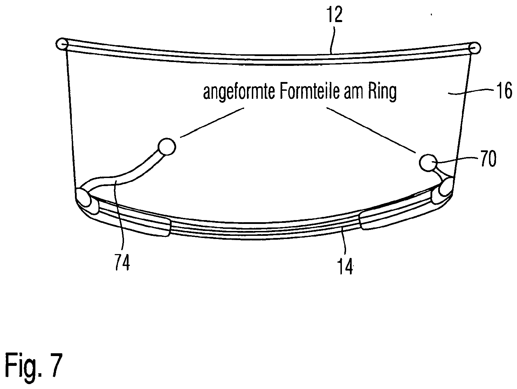

Bei der Ausführungsform gemäß Fig. 7 sind Spielkörper 74 und 76 an dem Vestibulärring 14 angebracht. Diese regen die Zunge an, sich mit ihr zu beschäftigen, so dass hierdurch gleichsam spielerisch die Zunge vom Behandlungsort ferngehalten wird.

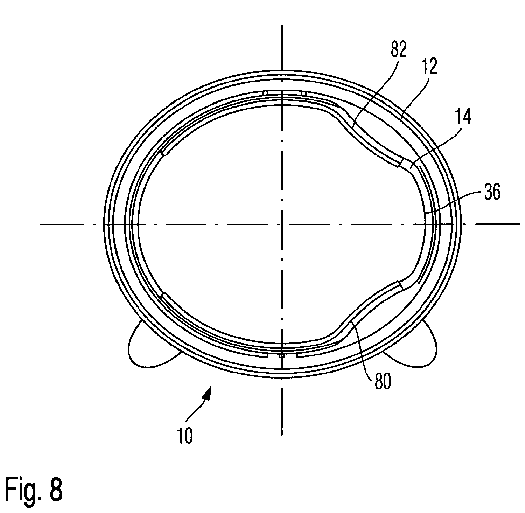

Wie aus Fig. 8 ersichtlich ist, ist es auch möglich, in dieser Ausgestaltung eine asymmetrische Realisierung des Vestibulärrings 14 vorzunehmen. Wenn beispielsweise bestimmte Bereiche des Mundes bzw. der Lippen eine geringere Öffnung erfordern, lässt sich eine Entspannung des Patienten hierdurch realisieren, dass dort der Vestibulärring 14 in radialer Richtung weiter zurückspringt. Der Patient empfindet eine langwierige Mundöffnung dann als weniger unangenehm, was weiter zur Compliance und Patientenverträglichkeit des erfindungsgemäßen Folienspannelements 10 beiträgt.

Hierzu weist der Vestibulärring kurzerhand Einbauchungen 80 und 82, in welchen er im Bereich der Polster, also von dem Lateralbereich 36 beabstandet, leicht radial einwärts durchgebogen ist.

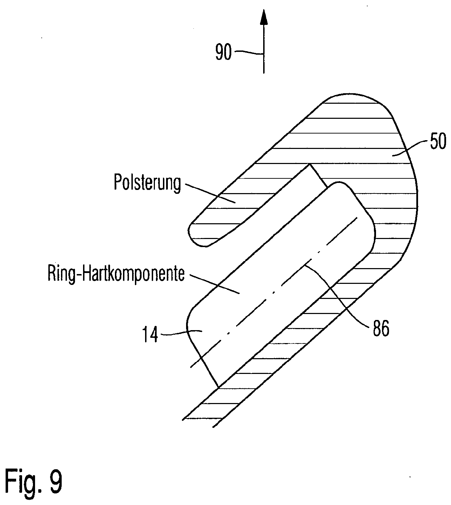

Aus Fig. 9 ist ersichtlich, dass der Vestibulärring 14 auch mit einem nicht kreisförmigen Querschnitt, beispielsweise bandförmig ausgestaltet sein kann. In dem dargestellten Ausführungsbeispiel erstreckt sich die Erstreckungsachse 86 des Vestibulärbandes schräg nach außen, und der Querschnitt des Vestibulärrings 14 ist im wesentlichen rechteckig mit abgerundeten Ecken. Er kann anstelle dessen auch trapezförmig sein, wobei stets wichtig ist, dass das Polster 50 die Ring-Hartkomponente 14 gerade nach radial auswärts (Pfeil 90) großvolumig umgibt.

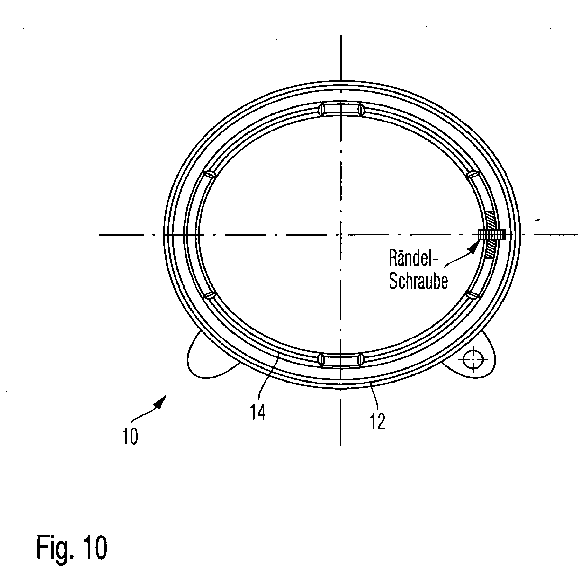

Gemäß einer weiteren Ausführungsform ist es vorgesehen, die Länge des Vestibulärrings 14 verstellbar zu machen. Hierdurch ist es möglich, mit dem gleichen Folienspannelement 10 zahlreiche unterschiedliche Patientengrößen abzudecken, beispielsweise auch Kinder. Die Verstellbarkeit kann in beliebiger geeigneter Weise realisiert sein, wobei in Fig. 10 beispielhaft eine Rändelschraube vorgesehen ist.

Anstelle dessen kann auch der Vestibulärring 16 in seiner Ringform einen Überlappungsbereich aufweisen, und die Enden des Vestibulärrings können gegeneinander verschieblich, aber fixierbar gelagert sein.

Es ist auch möglich, den Vestibulärring an einem Ende mit einem Raster und an dem andere Ende mit einer Rastzunge zu versehen, die je aufeinander zugewandt sind, um die Länge in beliebiger geeigneter Weise, jedoch fixierbar, einzustellen. Die Fixierung kann hierbei anstelle des Formschlusses bei Bedarf auch lediglich durch Reibschluß erfolgen.

## Patentansprüche

1. Folienspannelement, insbesondere für die Vornahme dentaler Bearbeitungen im Mundraum eines Patienten, mit einem Vestibulärring (14) und einem Lippenring (12), zwischen welchen und ggf. über welche hinaus sich eine Folie (16) erstreckt, die von den Ringen aufgespannt gehalten ist, wobei die Folie (16) und die Ringe unter Aufbringung von Kräften zwischen einer Oberseite des Folienspannelements und dessen Unterseite elastisch verformbar sind, wobei der Vestibulärring (14) durch Zusammendrücken an der Oberseite und an der dieser gegenüberliegenden Unterseite in eine im wesentlichen ovale Form zusammendrückbar ist, **dadurch gekennzeichnet, dass** der Vestibulärring (14) an mindestens einer Umfangsstelle, insbesondere der Oberseite und/oder der Unterseite, einen Rücksprung aufweist, dessen Breite größer als die Breite eines Frenulums und kleiner als der Radius des Vestibulärrings (14) ist, und dass ein Vorspannkorsett oder Lippenstützkorsett für die Folie (16) sich mindestens teilweise zwischen dem Vestibulärring (14) und dem Lippenring (12) erstreckt, das die Form der Folie (16) der Zielform in dem eingesetzten Zustand annähert.

2. Folienspannelement nach Anspruch 1, **dadurch gekennzeichnet, dass** mindestens ein Rücksprung ausgebildet ist, insbesondere zwei aneinander am Umfang gegenüberliegende Rücksprünge (18 und 20), die eine Breite von 0,5cm bis 3cm insbesondere etwa 1cm, aufweisen.

3. Folienspannelement nach einem der vorhergehenden Ansprüche, **dadurch gekennzeichnet, dass** der Vestibulärring (14) in seinem sich an den Rücksprung anschließenden Bereich einen insbesondere gepolsterten Vorsprung (26, 28, 30, 32) aufweist und dass die die ovale Form erzeugenden Kräfte im wesentlichen zwischen Den einander gegenüberliegenden Rücksprüngen (18,20) und Vorsprüngen (26, 28; 30,32) wirken und diese aufeinander zu drücken.

4. Folienspannelement nach einem der vorhergehenden Ansprüche, **dadurch gekennzeichnet, dass** der Vestibulärring (14) ein insbesondere auch plastisch verformbares Material aufweist, das bei Raumtemperatur elastisch und bei 37°C mindestens auch plastisch verformbar ist.

5. Folienspannelement nach einem der vorhergeheden Ansprüche, **dadurch gekennzeichnet, dass** der Vestibulärring (14) im trockenen Zustand ausschließlich oder nahezu ausschließlich elastisch verformbar und bei Feuchtigkeit insbesondere auch plastisch verformbar ist.

6. Folienspannelement nach einem der vorhergehenden Ansprüche, **dadurch gekennzeichnet, dass** der Vestibulärring (14) ein bei Feuchtigkeit quellfähiges Material aufweist.

7. Folienspannelement nach einem der vorhergehenden Ansprüche, **dadurch gekennzeichnet, dass** der Vestibulärring (14) eine Abdeckung mit einer Membran aufweist, die für Feuchtigkeit durchlässig ist und dass innerhalb der Membran im Bereich des Vestibulärrings (14) Feuchtigkeitsabsorbtionskörper angeordnet sind.

8. Folienspannelement nach einem der vorhergehenden Ansprüche, **dadurch gekennzeichnet, dass** der Vestibulärring (14) mindestens ein Licht- und/oder Wärmepolymerisierbares Material aufweist.

9. Folienspannelement nach einem der vorhergehenden Ansprüche, **dadurch gekennzeichnet, dass** der Vestibulärring (14) reversibel verformbar ist, insbesondere auch bei plastischer Verformung teilweise elastisch zurückfedert.

10. Folienspannelement nach einem der vorhergehenden Ansprüche, **dadurch gekennzeichnet, dass** der Vestibulärring (14) mindestens einen Hohlkörper (52) aufweist, der mit Luft befüllbar ist, insbesondere über ein Ventil und eine Handpumpe.

11. Folienspannelement nach einem der vorhergehenden Ansprüche, **dadurch gekennzeichnet, dass** der Vestibulärring (14) mit Flüssigkeit wie Wasser befüllbar ist und einen nachgiebigen Hohlkörper (52) nach der Art eines Wasserbetts aufweist.

12. Folienspannelement nach einem der vorhergehenden Ansprüche, **dadurch gekennzeichnet, dass** der Vestibulärring (14) eine Polsterung aufweist, die ein saugfähiges und bei Flüssigkeitsaufnahme quellfähiges Material aufweist.

13. Folienspannelement nach einem der vorhergehenden Ansprüche, **dadurch gekennzeichnet, dass** der Vestibulärring (14) eine Polsterung mit einem Schaum oder einem anderen nachgiebigen Material aufweist.

14. Folienspannelement nach einem der vorhergehenden Ansprüche, **dadurch gekennzeichnet, dass** der Vestibulärring (14) eine Polsterung aufweist, die mit einer Membran überzogen ist, welche Flüssigkeit von Außen nach Innen durchlässt und von Innen nach Außen sperrt.

15. Folienspannelement nach einem der vorhergehenden Ansprüche, **dadurch gekennzeichnet, dass** ein Zungenniederhalter an den Vestibulärring (14) angeformt ist, der sich von dem Vestibulärring (14) ausgehend von dem Folienspannelement (10) weg erstreckt.

16. Folienspannelement nach einem der vorhergehenden Ansprüche, **dadurch gekennzeichnet, dass** radial einwärts an der Folie (16) oder an dem Vestibulärring (14) oder dem Lippenring (12) Spielelemente für die Zunge des Patienten angebracht sind, die für die sensorische Attraktion der Zunge bestimmt sind.

17. Folienspannelement nach einem der vorhergehenden Ansprüche, **dadurch gekennzeichnet, dass** die Ringe, insbesondere der oval verformte Vestibulärring (14), asymmetrisch zur Verstärkung oder Abschwächung der nach radial auswärts wirkenden Spannkraft mindestens eine Abflachung oder einen Bogen an mindestens einer vorgegebenen Stelle aufweisen.

18. Folienspannelement nach einem der vorhergehenden Ansprüche, **dadurch gekennzeichnet, dass** der Vestibulärring (14) im Querschnitt unrund ausgebildet ist und insbesondere einen gegenüber der Ringachse schrägstehenden, im wesentlichen rechteckigen oder trapezförmigen Querschnitt mit abgerundeten Ecken aufweist, der insbesondere mindestens radial nach Außen gepolstert ist.

19. Folienspannelement nach einem der vorhergehenden Ansprüche, **dadurch gekennzeichnet, dass** eine Verdickung der Folie (16) als Polsterung des Vestibulärrings (14) ausgebildet ist.

20. Folienspannelement nach einem der vorhergehenden Ansprüche, **dadurch gekennzeichnet, dass** die Folie (16) gegenüber dem Vestibulärring (14) unverschiebbar gehalten ist und diesen insbesondere mindestens zur Hälfte, bevorzugt zu etwa drei Vierteln oder vollständig, umgibt.

21. Folienspannelement nach einem der vorhergehenden Ansprüche, **dadurch gekennzeichnet, dass** der Vestibulärring (14) bandförmig ausgebildet und dass sich die Längsachse des Bandes in einem Winkel von 45° oder weniger zur Achse (22) des Vestibulärrings (14) erstreckt und dass insbesondere das Band von einer Polsterung umgeben ist, die aus einem gegenüber dem eigentlichen Vestibulärring (14) weicheren Material besteht.

22. Folienspannelement nach einem der vorhergehenden Ansprüche, **dadurch gekennzeichnet, dass** der Vestibulärring (14) und insbesondere auch der Lippenring (12) eine Einstellvorrichtung aufweist, über welche seine Länge, und damit die Größe der von ihm aufgespannten Durchtrittsausnehmung, änderbar ist.

## Claims

1. A film clamping element, especially for performing dental processing in the oral cavity of a patient, having a vestibular ring (14) und a lip ring (12), between which and eventually beyond which a film (16) extends, which is held spanned by the rings, wherein the film (16) and the rings are elastically deformable by applying forces between a top side of the film clamping element and the bottom side of which is elastically deformable, wherein the vestibular ring (14) is compressible by compressing the top side and the bottom side opposite to It to an essentially oval shape, **characterized in that** the vestibular ring (14), at at least one circumferential location; especially the top side and/or the bottom side, comprises a recess, the width of which is larger than the width of a frenulum and smaller than the radius of the vestibular ring (14) and **In that** a biasing brace or a lip supporting brace for the film (16) extends at least partially between the vestibular ring (14) and the lip ring (12), the brace approximates the shape of the film (16) to the target shape in the inserted state.

2. The film clamping element according to Claim 1, **characterized in that** at least one recess is formed, especially two recesses (18 and 20) opposite to each other at the circumference, which have a width of 0.5cm to 3cm especially about 1cm.

3. The film clamping element according to one of the preceding Claims, **characterized in that** the vestibular ring (14) has an especially cushioned projection (26, 28, 30, 32) In Its region adjacent to the recess and **In that** the forces creating the oval shape essentially act between the recesses (18, 20) opposing each other and the projections (26, 28; 30,32) and to press them to each other.

4. The film clamping element according to one of the preceding Claims, **characterized in that** the vestibular ring (14) comprises a material especially also plastically deformable, which at room temperature is elastically deformable and at 37°C is at least also plastically deformable.

5. The film clamping element according to one of the preceding Claims, **characterized in that** the vestibular ring (14), In the dry state, is exclusively or almost exclusively elastically deformable and in the presence of humidity especially Is also plastically deformable.

6. The film clamping element according to one of the preceding Claims, **characterized in that** the vestibular ring (14) comprises a material swellable in the presence of humidity.

7. The film clamping element according to one of the preceding Claims, **characterized in that** the vestibular ring (14) comprises a cover with a membrane, which is permeable against humidity and **in that** humidity absorbing bodies are disposed within the membrane in the region of the vestibular ring (14).

8. The film clamping element according to one of the preceding Claims, **characterized In that** the vestibular ring (14) comprises at least one photo polymerizable and/or heat polymerizable material.

9. The film clamping element according to one of the preceding Claims, **characterized in that** the vestibular ring (14) is reversibly deformable, and especially when plastically deformed, also springs back elastically.

10. The film clamping element according to one of the preceding Claims, **characterized in that** the vestibular ring (14) comprises at least one hollow body (52), which may be filled with air, especially through a valve and a manual pump.

11. The film clamping element according to one of the preceding Claims, **characterized in that** the vestibular ring (14) may be filled with fluid, such as water and comprises a resilient hollow body (52) of the waterbed type.

12. The film clamping element according to one of the preceding Claims, **characterized in that** the vestibular ring (14) comprises a cushion, which comprises an absorbent and, during fluid intake, swellable material

13. The film clamping element according to one of the preceding Claims, **characterized in that** the vestibular ring (14) comprises a cushion with a foam or another swellable material.

14. The film clamping element according to one of the preceding Claims, **characterized in that** the vestibular ring (14) comprises a cushion coated with a membrane, which passes fluid from the exterior side to the inside and blocking it from the inside to the exterior side.

15. The film clamping element according to one of the preceding Claims, **characterized In that** a tongue compressor rod is shaped to fit to the vestibular ring (14), which extends starting from the vestibular ring (14) away from the film clamping element (10).

16. The film clamping element according to one of the preceding Claims, **characterized in that** play elements for the patient?s tongue are attached to the film (16) or the vestibular ring (14) or the lip ring (12) radially inwards, which are designed for the sensory attraction of the tongue.

17. The film clamping element according to one of the preceding Claims, **characterized in that** the rings, especially the ovally deformed vestibular ring (14), asymmetrically to the increase or decrease of the clamping force acting radially outwards, has at least one flattening or an arc at least at one predetermined location.

18. The film clamping element according to one of the preceding Claims, **characterized in that** the vestibular ring (14), in cross section, is formed non-circular and especially comprises an essentially rectangular or trapezoidal cross-section with rounded corners, the cross section being inclined in relation to the ring axis and being cushioned at least In the radial outward direction.

19. The film clamping element according to one of the preceding Claims, **characterized In that** a thickening of the film (16) Is formed as a cushion of the vestibular ring (14).

20. The film clamping element according to one of the preceding Claims, **characterized in that** the film (16) is non-displaceably maintained in relation to the vestibular ring (14) and surrounding it especially at least halfway, preferably to about three-quarters or completely.

21. The film clamping element according to one of the preceding Claims, **characterized in that** the vestibular ring (14) is formed In a ribbon-shaped manner and **in that** the longitudinal axis of the ribbon extends in an angle 45° or less in relation to the axis (22) of the vestibular ring (14) and **in that** especially the ribbon is surrounded by a cushioning, which consists of material which is softer as compared to the actual vestibular ring (14).

22. The film clamping element according to one of the preceding Claims, **characterized In that** the vestibular ring (14) and especially also the lip ring (12) comprises a setting device, by mans of which the length thereof, and thus the size of the passage recess spanned by it may be changed.

## Revendications

1. Élément de tension pour films, en particulier pour effectuer des soins dentaires dans la bouche d'un patient, avec une bague vestibulaire (14) et une bague des lèvres (12), entre lesquelles et éventuellement au-delà desquelles s'étend un film (16), qui est maintenue tendu par les bagues, où le film (16) et les bagues sont déformables élastiquement sous l'application de forces entre une face supérieure de l'élément de tension pour films et sa face inférieure, où la bague vestibulaire (14) peut être comprimée en une forme essentiellement ovale par la compression de sa face supérieure et de sa face inférieure en face de celle-ci, **caractérisé en ce que** la bague vestibulaire (14) présente un enfoncement à au moins un endroit de sa circonférence, en particulier à sa surface supérieure et/ou sa surface inférieure, dont la largeur (14) est supérieure à la largeur d'un frenulum et plus petit que le rayon de la bague vestibulaire, et qu'un corset de pré-tension ou de soutien des lèvres pour le film (16) s'étend au moins partiellement entre la bague vestibulaire (14) et la bague des lèvres (12), et rapproche la forme du film (16) à la forme ciblée à l'état montée.

2. Élément de tension pour films selon la revendication 1, **caractérisé en ce qu'**au moins un enfoncement est formé, en particulier deux enfoncements en face à face sur la périphérie (18 et 20), qui présentent une largeur de 0, 5 cm à 3 cm, en particulier environ 1 cm.

3. Élément de tension selon l'une des revendications précédentes, **caractérisé en ce que** la bague vestibulaire (14) présente une protubérance en particulier rembourrée (26, 28, 30, 32) dans la région adjacente à l'enfoncement et que les forces générant la forme ovale agissent essentiellement entre les enfoncements (18, 20) et les protubérances (26, 28 ; 30,32), les uns en face des autres, et pour les appuyer les uns sur les autres.

4. Élément de tension selon l'une des revendications précédentes, **caractérisé en ce que** la bague vestibulaire(14) présente un matériau déformable, en particulier plastiquement, qui est élastique à la température ambiante et est aussi plastiquement déformable à au moins 37°C.

5. Élément de tension selon l'une des revendications précédentes, **caractérisé en ce que** la bague vestibulaire(14) à l'état sec est exclusivement ou presque exclusivement élastiquement déformable et en particulier aussi plastiquement déformable en présence de l'humidité.

6. Élément de tension selon l'une des revendications précédentes, **caractérisé en ce que** la bague vestibulaire(14) présente un matériel absorbant en présence de l'humidité.

7. Élément de tension selon l'une des revendications précédentes, **caractérisé en ce que** la bague vestibulaire (14) présente une couverture avec une membrane, qui est perméable à l'humidité et que des corps d'absorption de l'humidité sont disposés au sein de la membrane dans le domaine de la bague vestibulaire (14).

8. Élément de tension selon l'une des revendications précédentes, **caractérisé en ce que** la bague vestibulaire (14) présente au moins un matériel photo- et/ou thermopoly-mérisable.

9. Élément de tension selon l'une des revendications précédentes, **caractérisé en ce que** la bague vestibulaire (14) peut être déformée de manière réversible, en particulier qu'elle rebondit partiellement élastiquement même en cas de déformation plastique.

10. Élément de tension selon l'une des revendications précédentes, **caractérisé en ce que** la bague vestibulaire 14) présente au moins un corps creux (52), qui peut être rempli d'air, en particulier par une vanne et une pompe manuelle.

11. Élément de tension selon l'une des revendications précédentes, **caractérisé en ce que** la bague vestibulaire(14) peut être remplie d'un liquide comme de l'eau et présente un corps creux souple (52) à la manière d'un lit d'eau.

12. Élément de tension selon l'une des revendications précédentes, **caractérisé en ce que** la bague vestibulaire(14) présente un rembourrage, qui présente un matériau absorbant capable de gonfler quand il absorbe du liquide.

13. Élément de tension selon l'une des revendications précédentes, **caractérisé en ce que** la bague vestibulaire(14) présente un rembourrage avec une mousse ou un autre matériel souple.

14. Élément de tension selon l'une des revendications précédentes, **caractérisé en ce que** la bague vestibulaire(14) présente un rembourrage qui est recouvert d'une membrane qui laisse passer un liquide de l'extérieur vers l'intérieur et se verrouille de l'intérieur vers l'extérieur.

15. Élément de tension selon l'une des revendications précédentes, **caractérisé en ce qu'**un dispositif de retenue de la langue est formé sur la bague vestibulaire (14), qui, en partant depuis la bague vestibulaire (14) s'éloigne de l'élément de tension (10).

16. Élément de tension selon l'une des revendications précédentes, **caractérisé en ce que** des éléments pour la langue du patient sont installés radialement vers l'intérieur sur le film (16) ou la bague vestibulaire (14) ou la bague de lèvre (12), qui sont destinés à l'attraction sensorielle de la langue.

17. Élément de tension selon l'une des revendications précédentes, **caractérisé en ce que** les bagues, en particulier la bague vestibulaire (14) ovale présente un méplat ou un arc à au moins une position prédéterminée, pour le renforcement ou l'affaiblissement asymétrique de la force de torsion agissant radialement vers l'extérieur.

18. Élément de tension selon l'une des revendications précédentes, **caractérisé en ce que** la bague vestibulaire (14) n'est pas ronde dans la coupe transversale et présente en particulier une coupe transversale oblique par rapport à l'axe annulaire, essentiellement rectangulaire ou trapézoïdale avec des coins arrondis, qui en particulier est rembourrée au moins radialement vers l'extérieur.

19. Élément de tension selon l'une des revendications précédentes, **caractérisé en ce qu'**un épaississement du film (16)) est formé comme rembourrage de la bague vestibulaire (14).

20. Élément de tension selon l'une des revendications précédentes, **caractérisé en ce que** le film (16) est maintenu fixe par rapport à la bague vestibulaire(14) et entoure celui-ci au moins à moitié, de préférence au moins à environ trois quarts ou entièrement.

21. Élément de tension selon l'une des revendications précédentes, **caractérisé en ce que** la bague vestibulaire (14) est formée sous forme de bande et que l'axe longitudinal de la bande s'étend à un angle de 45° ou moins par rapport à l'axe (22) de la bague vestibulaire (14) et qu'en particulier la bande (14) est entourée d'un rembourrage qui est composé d'un matériau plus souple par rapport à la bague vestibulaire en soi.

22. Élément de tension selon l'une des revendications précédentes, **caractérisé en ce que** la bague vestibulaire(14), et en particulier aussi la bague de lèvre (12) comportent un dispositif de réglage, par lequel la longueur et par conséquent la taille de la cavité de passage tendue par leur biais peuvent être modifiés.
